Europäisches Patentamt

European Patent Office

Office européen des brevets

(19)

(11) **EP 0 687 173 B1**

(12) **FASCICULE DE BREVET EUROPEEN**

(45) Date de publication et mention
de la délivrance du brevet:
**17.09.1997 Bulletin 1997/38**

(21) Numéro de dépôt: **94908391.9**

(22) Date de dépôt: **01.03.1994**

(51) Int. Cl.$^6$: **A61K 9/50**, A61K 9/16,
A61K 9/51, A61K 7/00

(86) Numéro de dépôt international:
**PCT/FR94/00228**

(87) Numéro de publication internationale:
**WO 94/20078 (15.09.1994 Gazette 1994/21)**

(54) **VECTEURS PARTICULAIRES SYNTHETIQUES ET PROCEDE DE PREPARATION**

TEILCHENFOERMIGE SYNTHETISCHE TRAEGER SOWIE VERFAHREN ZU DEREN
HERSTELLUNG

SYNTHETIC PARTICULATE VECTORS AND METHOD FOR PREPARING SAME

(84) Etats contractants désignés:
**AT BE CH DE DK ES FR GB GR IE IT LI LU MC NL
PT SE**

(30) Priorité: **02.03.1993 FR 9302397**

(43) Date de publication de la demande:
**20.12.1995 Bulletin 1995/51**

(60) Demande divisionnaire:**97102586.1
97104152.0**

(73) Titulaire: **BIOVECTOR THERAPEUTICS SA
31676 Labege (FR)**

(72) Inventeurs:
• **SAMAIN, Daniel
F-31400 Toulouse (FR)**
• **DELRIEU, Pascal
F-81000 Albi (FR)**
• **GIBILARO, Jo[lle
F-37000 Tours (FR)**
• **DIRSON, Roselyne
F-33550 Capian (FR)**

• **CERVILLA, Monique
F-31400 Toulouse (FR)**
• **DE MIGUEL, Ignacio
F-31400 Toulouse (FR)**
• **DING, Li
F-31400 Toulouse (FR)**
• **NGUYEN, Frédérique
F-17640 Vaux-sur-Mer (FR)**
• **SOULET, Nadine
F-11410 Salles-sur-l'Hers (FR)**
• **SOLER, Corinne
F-66140 Canet-en-Roussillon (FR)**

(74) Mandataire: **Warcoin, Jacques et al
Cabinet Régimbeau,
26, avenue Kléber
75116 Paris (FR)**

(56) Documents cités:
**EP-A- 0 277 776**     **EP-A- 0 344 040**
**WO-A-91/16068**     **WO-A-92/21329**

Remarques:
Demande divisionnaire 97102586.1 déposée le
17/02/97.

EP 0 687 173 B1

**Description**

La présente invention concerne de nouveaux types de particules, utilisables seuls ou comme vecteurs de différents composés. Elle se rapporte également à un procédé de préparation de vecteurs particulaires permettant un contrôle amélioré du chargement en principe actif.

Les BIOVECTEURS SUPRAMOLÉCULAIRES ou BVSM sont des particules, biomimétiques des vecteurs endogènes de l'organisme, capables d'encapsuler et de vectoriser un grand nombre de principes actifs pour une utilisation notamment pharmaceutique, cosmétique ou agroalimentaire.

Un premier type de BVSM a été décrit dans la demande EP 344 040. Leur structure est très bien adaptée au rôle de vecteur, notamment du fait de la possibilité de modifier leur taille et leur composition suivant la ou les molécules transportées et leur utilisation.

Les BVSM sont synthétisés en trois étapes successives:

-   synthèse d'un noyau central constitué par exemple de polysaccharide naturel réticulé, pouvant être dérivé par des groupements ioniques, et ramené, notamment par ultrabroyage, à la taille désirée (entre 10 nanomètres et quelques microns suivant l'utilisation désirée)
-   établissement d'une couronne d'acides gras greffés de façon covalente uniquement à la périphérie du noyau central, afin de conférer à celui-ci un caractère hydrophobe périphérique tout en conservant son caractère hydrophile interne
-   stabilisation d'un ou de plusieurs feuillets lipidiques externes, constitués notamment de phospholipides ou de céramides, parfois additionné d'autres constituants, par exemples des constituants des membranes biologiques.

Les principes actifs, suivant leurs caractéristiques physicochimiques, peuvent être transportés soit dans les feuillets lipidiques externes (cas de composés lipophiles ou amphiphiles) soit à l'intérieur du noyau hydrophile (cas des composés polaires).

L'encapsulation des principes actifs de nature polaire peut se dérouler, suivant la structure de celui-ci, soit avant l'établissement de la couronne d'acides gras, soit entre cette étape et la stabilisation du feuillet externe.

Malgré leur adaptabilité à de nombreuses utilisations, la synthèse des BVSM peut parfois poser des problèmes et notamment:

-   elle exige une étape délicate à maîtriser pour greffer la couronne d'acides gras;
-   ce greffage, uniquement réalisé en périphérie du noyau, doit être effectué de façon homogène, ce qui nécessite notamment une étape de séchage préalable, dans des conditions très spécifiques;
-   si le principe actif est encapsulé avant le greffage de la couronne d'acides gras, certaines de ces molécules, localisées, après leur encapsulation, à la périphérie du noyau, pourront être dérivées par l'acide gras, conduisant à la modification des propriétés de ce principe actif;
-   si le principe actif est encapsulé après le greffage de la couronne d'acides gras, celle-ci peut représenter une gène pour la pénétration du principe actif dans le noyau hydrophile.

La Demanderesse a montré que, de manière surprenante, pour certaines applications, il était possible de réduire le schéma réactionnel en ne greffant pas la couronne d'acides gras à la périphérie du noyau hydrophile maillé.

La Demanderesse a montré que les particules polysaccharidiques ainsi obtenues pouvaient être utilisées telles quelles. Elles sont alors nommées, par analogie avec les Biovecteurs Supramoléculaires, BVSM de type-PS.

La Demanderesse a en effet montré que les particules polysaccharidiques, même de petite taille, pouvaient être utilisées moyennant l'adoption de protocoles de chargement adaptés.

Elles peuvent également être utilisées en association avec des composés amphiphiles naturels, notamment des phospholipides et la Demanderesse a montré que les feuillets lipidiques externes ont la possibilité de s'organiser autour de ce noyau en l'absence d'une couronne lipidique greffée comme dans le cas des BVSM. Ces particules possèdent un caractère supramoléculaire et sont appelés BVSM type-L.

C'est pourquoi la présente invention à pour objet un vecteur particulaire synthétique, caractérisé en ce qu'il comporte :

-   un noyau hydrophile non liquide,
-   une couche externe constituée au moins en partie soit de composés amphiphiles, associée au noyau par des interactions hydrophobes et/ou des liaisons ioniques, soit par la couronne externe du noyau hydrophile, en utilisant un procédé spécial évitant l'encapsulation du principe actif dans cette couronne et concentrant le principe actif dans la partie interne du noyau.

La notion de vecteur doit ici être entendue au sens large, c'est-à-dire qu'elle comprend des particules ayant un rôle de support, par exemple quand elles sont incorporées dans une composition, soit telles quelles, soit pour le transport, la présentation et/ou la stabilisation de composés actifs.

Le noyau (ou matrice) hydrophile non liquide peut être un polymère hydrophile. La matrice hydrophile peut notamment être constituée de polysaccharides ou d'oligosaccharides, naturellement ou chimiquement réticulés. De préférence, le polysaccharide est choisi parmi le dextrane, l'amidon, la cellulose et leurs dérivés.

Deux types d'interactions peuvent expliquer la stabilisation des phospholipides par exemple sur un noyau constitué de polysaccharide réticulé et dérivé dans sa masse par des groupements ioniques: ce sont soit des interactions de type hydrophobe, soit des liaisons de type ionique, les deux modes pouvant coopérer à la stabilisation de ces feuillets.

En effet, les phospholipides sont constitués, sur la base d'un motif glycérol, d'une tête polaire comprenant un groupement phosphate, de charge anionique forte, éventuellement dérivé par des groupements polaires divers et de deux acides gras constituant la queue hydrophobe.

La tête polaire possède la capacité de se lier par interaction ionique avec les groupements ioniques greffés dans la matrice polysaccharidique, soit par l'intermédiaire du phosphate, soit par l'intermédiaire des groupements ioniques greffés sur le phosphate du phospholipide (ammonium quaternaire des phosphatidylcholines par exemple).

D'autre part, on sait que les polysaccharides, s'ils ont un caractère globalement hydrophile, possèdent un sillon hydrophobe, établi par le fait que les groupements hydroxyles polaires s'orientent dans une direction donnée, laissant accessible la structure de base des sucres, constituée de liaisons carbone-carbone, de nature hydrophobe.

Dans le cas des particules polysaccharidiques constituant le noyau des BVSM type-L, la stabilisation de composés comme les phospholipides peut être due à un phénomène de coopérativité entre les deux modes de liaisons.

L'association phospholipides-noyaux polysaccharidiques a pu être démontrée par des techniques de transfert d'énergie en fluorescence. La théorie du transfert d'énergie repose sur les interactions qui existent entre deux fluorophores lorsque la bande d'émission du premier fluorophore (F1), recouvre la bande d'excitation du second fluorophore (F2). Si les deux éléments sont proches l'énergie d'un photon absorbée par le fluorophore F1 peut être transférée au fluorophore F2 qui va alors fluorescer aussi bien que s'il avait été excité directement. La fluorescence de F1 peut alors diminuer jusqu'à extinction totale. L'efficacité du transfert d'énergie entre les deux fluorophores est donc dépendante de leur séparation spatiale. Après avoir effectué un marquage à l'aide de l'isothiocyanate de Rhodamine sur les noyaux polysacharidiques et à l'aide du Nitrobenzodiazole (NBD) sur les phospholipides un transfert d'énergie a pu être mis en évidence entre les deux fluorophores que ce soit dans le cas des BVSM de type-L de 1 $\mu$m, 200nm ou de 20nm. Ce transfert reste stable après des incubations à 4°C, 37°C et même à 100°C.

Le noyau hydrophile peut être obtenu par différentes méthodes et en particulier, s'il s'agit d'un noyau de nature polysaccharidique, en utilisant un polysaccharide biodégradable, ramifié ou linéaire. Ce polysaccharide peut être par exemple de l'amidon ou l'un de ses dérivés. Les procédés de réticulation sont connus de l'homme de l'art et peuvent être effectués au moyen d'agents bi ou tri-fonctionnels, comme l'épichlorhydrine ou l'oxychlorure de phosphore.

Les propriétés du polysaccharide peuvent être modifiées en substituant les sucres par des fonctions ioniques, acides ou basiques, qui sont importantes dans la stabilisation du feuillet lipidique externe et pour l'encapsulation de principes actifs ioniques.

L'encapsulation des principes actifs hydrophiles peut être réalisée à ce stade de la synthèse. Le gel obtenu lors de l'étape de synthèse est alors lavé et déshydraté partiellement au moyen par exemple de techniques de centrifugation, puis mis en présence du principe actif et réhydraté lentement. Le gel ayant la capacité de se gonfler d'eau, l'actif est entrainé à l'intérieur du réseau polysaccharidique où il peut se lier par des liaisons ioniques avec les groupements greffés à l'intérieur du gel.

Le gel obtenu, qu'il contienne ou non un composé encapsulé, doit être broyé mécaniquement en vue d'obtenir des particules de taille désirée. Les méthodes d'ultrabroyage sont connues dans l'état de la technique et peuvent notamment faire appel à une extrusion à haute pression au moyen d'un homogénéisateur.

L'encapsulation de composés hydrophiles à l'intérieur des BVSM de type-L est généralement réalisée à cette étape. Pour celà, la suspension de particules est préalablement séchée en utilisant des méthodes de séchage, comme la lyophilisation ou l'atomisation.

Les particules séchées sont par exemple mélangées avec le principe actif également sous forme sèche. Une réhydratation progressive permet, comme dans le cas du gel, de solubiliser le principe actif puis de l'entrainer à l'intérieur de la particule où il se lie par liaisons ioniques.

Le feuillet lipidique externe des BVSM de type-L peut être réalisé avec différents types de lipides naturels ou synthétiques, parmi lesquels les phospholipides et les céramides, mais aussi des tensio-actifs ioniques ou non-ioniques capables de s'organiser en micelles, auxquels peuvent également être additionnés d'autres composés soit lipidiques soit amphiphiles comme du cholestérol, des acides gras ou des vitamines liposolubles.

Ce feuillet est obtenu de préférence en utilisant des méhodes qui servent à la préparation des liposomes, c'est-à-dire la préparation en phase inverse, la dialyse de détergent ou l'extrusion à haute pression. Les principes actifs devant être vectorisés ou présentés à la surface des BVSM de type-L peuvent être introduits lors de cette étape, mélangés aux tensio-actifs.

La présente invention a également pour objet un procédé de préparation d'un vecteur particulaire, caractérisé en ce que :

a) on effectue l'encapsulation d'au moins un principe actif ionisable basique, dans une matrice hydrophile réticulée greffée par des ligands acides, à un pH inférieur au $pK_a$ du principe actif,

b) on augmente le pH du milieu jusqu'à une valeur supérieure au $pK_a$ du principe actif,

c) on récupère la matrice hydrophile comportant le principe actif localisé principalement au centre de ladite matrice.

En effet, l'adoption d'un protocole de chargement de noyaux hydrophiles adapté, permet le contrôle topologique du chargement.

La matrice hydrophile est de préférence constituée de polysaccharides ou d'oligosaccharides, naturellement ou chimiquement réticulés.

Ce procédé qui peut être utilisé avec les BVSM est plus particulièrement important avec des particules dont les feuillets lipidiques externes ont été réduits (BVSM de type-L) ou supprimés (BVSM de type-PS) par rapport au modèle précédemment décrit. La Demanderesse a observé qu'il est difficile d'utiliser de tels BVSM à feuillets lipidiques réduits comme vecteurs pour l'encapsulation de principes actifs ioniques, avec des méthodes de chargement classiques.

En effet, si des molécules du principe actif se lient avec la particule polysaccharidique du noyau tout en se maintenant à la périphérie de celui-ci, il peut en résulter une instabilité de la suspension de particules, celles-ci pouvant s'agréger entre-elles grâce à des liaisons interparticulaires dues au principe actif. Ce phénomène est relativement mineur pour de faibles taux de chargement en principes actifs, alors qu'il devient très important avec taux de chargement en principes actifs élevés. De même, la taille des particules est extrêmement importante. Avec des particules de grande taille (ex. supérieur à 100 nanomètres), le rapport entre la surface et le volume interne de la particule est très faible; de ce fait, en comparaison avec la quantité totale de principe actif encapsulé, la quantité de principe actif lié en périphérie des particules est très faible, limitant ainsi les possibilités de liaisons interparticulaires. A l'inverse, lorsque les particules possèdent une taille très faible, ce phénomène d'agrégation est très sensible. Il convient également de noter que ce phénomène est peu marqué avec des BVSM possédant une couche d'acides gras greffée sur le noyau, qui sert alors d'isolant aux interactions interparticulaires.

Pour remédier à cela, la Demanderesse a montré qu'il est possible de contrôler topologiquement la pénétration du principe actif à l'intérieur des particules par la maîtrise des conditions ioniques de l'encapsulation.

Les noyaux polysaccharidiques peuvent être considérés comme des matrices polyélectrolytes et, en tant que tels, ils présentent un différentiel de pH entre l'intérieur et l'extérieur de la particule. Ce phénomène est dû à la dissociation plus ou moins importante des contreions et à l'immobilisation des fonctions ioniques sur le réseau polysaccharidique. Cette propriété permet de contrôler la localisation du principe actif à incorporer, en forçant une encapsulation uniquement interne ou uniquement en surface ou encore dans la périphérie du noyau.

Lorsque le principe actif à incorporer est de nature basique, sa solubilisation dans un milieu de pH inférieur à son pKa le conduit à se présenter sous forme ionisée; il peut alors s'ancrer avec les groupements anioniques greffés sur le noyau polysaccharidique. Lorsque le pH remonte au dessus du pKa, le principe actif est sous une forme déionisée, qui ne lui permet pas d'interagir avec la matrice. Nous utilisons donc, pour contrôler la localisation du principe actif encapsulé, le différentiel de pH existant entre l'intérieur et l'extérieur de la particule: si le milieu extérieur présente un pH trop élevé, le principe actif ne peut pas interagir avec les groupements ioniques placés en périphérie des noyaux. Le pH interne des noyaux dérivés par des ligands acides étant plus bas que le pH externe, le principe actif, entré dans la particule sous forme déionisée, redevient ionique et se lie donc avec les groupements anioniques du BVSM type-L. Dans ce cas précis, le principe actif sera localisé uniquement dans le coeur de la particule, à l'exclusion de la zone périphérique. Ce type d'encapsulation est donc très favorable à une dispersion optimale des particules.

Dans le cas des principes actifs acides, on peut appliquer le procédé avec des noyaux dérivés par des ligands basiques, de manière symétrique, selon les étapes suivantes :

a) on effectue l'encapsulation d'au moins un principe actif ionisable acide, dans une matrice hydrophile réticulée greffée par des ligands ioniques basiques, à un pH supérieur au $pK_a$ du principe actif,

b) on diminue le pH du milieu jusqu'à une valeur inférieure au $pK_a$ du principe actif,

c) on récupère la matrice hydrophile comportant le principe actif localisé principalement au centre de ladite matrice.

Ce type de chargement avec contrôle topologique de la localisation du principe actif dans le noyau polysaccharidique est particulièrement intéressant pour des applications de vectorisation avec des BVSM dont les feuillets lipidiques externes ont été réduits (BVSM de type-L) ou supprimés (BVSM de type-PS), mais il est également adaptable et souhaitable pour des BVSM déjà décrits dans les brevets précédents afin d'augmenter le taux de chargement ou de minimiser les perturbations apportées à la structure du feuillet externe phospholipidique dans le cas de l'ancrage externe de macromolécules, par exemple de motifs de reconnaissance et notamment d'une apoprotéine.

L'invention a également pour objet un vecteur particulier constitué d'un noyau hydrophile réticulé, greffé par des groupements ioniques, appelé ici BVSM de type-PS. Les groupements ioniques peuvent être des groupements anioniques, comme par exemple phosphates succinates, carboxyméthylates, ou des groupements cationiques, par exemple des ammoniums quaternaires ou des amines. De préférence, la taille des BVSM type-PS est comprise entre 20 nm et 200 nm.

Le noyau hydrophile réticulé peut être constitué de polymères naturels ou synthétiques, naturellement ou chimiquement réticulés. On utilise notamment les polysaccharides ou oligosaccharides, tels que l'amidon, le dextranne, la cellulose et leurs dérivés.

De manière avantageuse, un principe actif est encapsulé dans le BVSM type-PS principalement au centre de la matrice ; la partie externe du noyau est pratiquement dépourvue de principe actif, ce qui permet d'éviter les phénomènes d'agrégation survenant généralement pour des particules de petite taille.

L'un des objets de l'invention est donc un vecteur particulier constitué d'une matrice polysaccharidique réticulée contenant un principe actif, ledit principe actif étant de préférence localisé principalement au centre de la matrice.

Selon encore un autre aspect, l'invention a pour objet un procédé de chargement, qui permet l'encapsulation du principe actif dans un BVSM complet, un BVSM de type-PS ou un BVSM de type-L, qui peuvent être sous forme de suspension.

Le chargement s'effectue sur le vecteur particulier sur lequel sont fixées, le cas échéant les différentes couches lipidiques et/ou amphiphiles. Pour cela, le noyau hydrophile doit comporter des groupements ioniques. Le procédé nécessite donc les étapes suivantes :

a) on prépare un noyau hydrophile réticulé dans lequel sont fixés des groupements ioniques,
b) de manière facultative, on fixe des composés lipidiques, tels par exemple des acides gras, sur le noyau réticulé et/ou, on établit un feuillet amphiphile (par exemple, phospholipidique) sur le noyau, éventuellement acylé,
c) on charge le principe actif à l'intérieur du vecteur à un pH adapté au principe actif et en fournissant de l'énergie,
d) on récupère le vecteur ayant incorporé le principe actif.

Dans le cas des BVSM de type PS, il est difficile d'utiliser les méthodes de chargement classiques pour incorporer les principes actifs ioniques. Il est vrai que les méthodes avec contrôle topologique permettent de pallier ce problème. Cependant, le contrôle topologique nécessite un jeu fin de pH qui doit être compatible avec le principe actif et le vecteur.

Dans le cas des BVSM normaux ou de type-L, les méthodes développées jusqu'à maintenant consistent à incorporer le principe actif dans les noyaux et à établir ensuite le feuillet phospholipidique. Celles-ci présentent certes des résultats d'incorporation très intéressants mais nécessitent la manipulation du principe actif à toutes les étapes du procédé de préparation. Dans le cas de principes actifs très toxiques ou très chers, ces méthodes doivent être déconseillées pour des raisons de sécurité ou de coût.

La demanderesse a donc développé une méthode alternative pour pallier ces problèmes. Les ligands ioniques greffés dans le réseau polysaccharidique des vecteurs induisent une affinité importante pour les principes actifs ioniques de charge opposée. Cependant, cette affinité, lors de l'incorporation, doit être contrôlée pour éviter l'agrégation des vecteurs et permettre la localisation du principe actif principalement à l'intérieur des particules. Pour le préchargement, ce contrôle nécessite un jeu fin de pH ou un taux d'incorporation peu élevé. Cette agrégation est dûe en majeure partie à la localisation en surface du principe actif qui elle-même est dûe à la présence de ligands en surface des particules.

Pour mettre au point ce nouveau type de chargement, trois facteurs entrent en jeu :

a) une affinité importante du principe actif pour le vecteur pour assurer l'incorporation du principe actif : cette affinité est créée par les ligands ioniques, acides ou basiques, qui sont greffés dans le polymère réticulé ; la densité et la force des ligands peuvent être ajustées en fonction du principe actif,
b) une dispersion importante des vecteurs lors de l'incorporation pour éviter les interactions entre particules qui favorisent l'agrégation : cette dispersion peut être assurée par la dilution des vecteurs dans le milieu réactionnel à une concentration suffisante pour diminuer les interactions interparticulaires mais aussi à une concentration compatible avec des applications pharmaceutiques,
c) la mise en oeuvre de tout moyen pour favoriser l'entrée du principe actif à l'intérieur du vecteur : l'apport d'énergie, sous forme par exemple d'agitation ou de chaleur, va accélérer la cinétique d'entrée du principe actif mais aussi favoriser la dispersion des vecteurs ; la forme adéquate du principe actif qui doit être suffisamment ionique pour permettre l'ancrage du principe actif mais aussi le moins chargé pour éviter les interactions superficielles.

Pour les BVSM, les BVSM de type L ou de type PS, il est possible de mettre en oeuvre des protocoles d'incorporation répondant à ces exigences. La présence des ligands ioniques greffés dans lepolymère réticulé assure l'ancrage des principes actifs pour les trois espèces. La dispersion des vecteurs peut être réalisée par la mise en suspension dans l'eau des BVSM de type PS. Les BVSM ou les BVSM de type L sont préparés à partir des noyaux, acylés ou polysaccharidiques, et de phospholipides, préalablement dispersés en milieu aqueux et se retrouvent donc en suspension dans l'eau. L'apport d'énergie, par exemple sous forme d'agitation ou de chaleur, n'est pas dégradant pour les BVSM. Il est possible de jouer avec les pH et de définir des plages de pH compatibles avec ce type de chargement.

Dans le cas des BVSM et des BVSM de type L, les feuillets lipidiques et/ou phospholipidiques représentent une barrière qui doit être franchie par les principes actifs. Mais, dans le procédé de chargement décrit, deux facteurs supplémentaires interviennent pour favoriser ce franchissement :

a) l'apport d'énergie qui peut fluidifier les feuillets lipidiques et phospholipidiques et augmenter les cinétiques d'entrée du principe actif à l'intérieur des vecteurs,

b) la forme du principe actif qui peut être très faiblement ionique du fait de l'existence du gradient existant entre l'intérieur et l'extérieur du vecteur.

En outre la forte affinité entre le principe actif et le polymère réticulé polyélectrolyte assure son ancrage et la forte dispersion évite l'agrégation des vecteurs entre eux.

Ce nouveau procédé permet de préparer les BVSM, de tout type, chargés en principe actif en conservant la taille des vecteurs de base et présente de nombreux avantages. Cette méthode consiste à préparer les vecteurs blancs, sans principe actif, avant l'incorporation, ce qui permet de processer les vecteurs blancs selon des conditions adaptées aux vecteurs et ne dépendant pas du principe actif à encapsuler et de charger ensuite. Ces conditions peuvent donc être plus ou moins drastiques. Elle permet aussi de pouvoir caractériser le vecteur blanc comme entité de base.

L'étape d'incorporation est l'étape finale du procédé, ce qui conduit à manipuler le principe actif, susceptible d'être toxique et coûteux, pendant seulement une étape lors du procédé. Ce procédé réduit donc les manipulations et les pertes éventuelles du principe actif. Il permet donc d'être plus sûr au niveau sécurité mais aussi plus rentable.

En outre, pour certains principes actifs, les conditions d'incorporation pourront être relativement simples, ce qui permet d'envisager de charger les vecteurs avec le principe actif de manière extemporanée. Ce mode de préparation extemporané peut éliminer les problèmes de conservation à l'état liquide.

Ce nouveau mode de chargement est basé sur l'importante affinité entre les vecteurs et les principes actifs ioniques mais aussi sur le contrôle simple de l'incorporation par la dispersion des vecteurs et la forme ionique du principe actif. Il présente des avantages très intéressants : préparation du vecteur blanc indépendante du principe actif, manipulation du principe actif à une seule étape, possibilité de préparation extemporanée.

L'invention a également pour objet un vecteur particulaire qui comporte de l'intérieur vers l'extérieur, une matrice polysaccharidique réticulée contenant un principe actif ionisable, une première couche lipidique fixée à la matrice par des liaisons covalentes, et une seconde couche de composés amphiphiles sur laquelle sont éventuellement greffés des molécules protéiques ou peptidiques.

Les vecteurs particulaires selon l'invention ont de préférence un diamètre compris entre 10nm et 5$\mu$m, de manière encore préférée entre 20 et 70 nm.

Ces vecteurs particulaires sont destinés à véhiculer ou à présenter à leur surface une ou plusieurs molécules à activité biologique. Parmi ces molécules, il faut citer, sans que cette liste soit limitative :

- les antibiotiques et les anti-viraux,
- les protéines, les protéoglycanes, les peptides,
- les polysaccharides, les lipopolysaccharides,
- les anticorps,
- les antigènes,
- les insecticides et fongicides,
- les composés agissant sur le système cardio-vasculaire,
- les anti-cancéreux,
- les anti-malariques,
- les anti-asthmatiques,
- les composés présentant une action sur la peau,
- les constituants des globules gras laitiers.

Dans les exemples ci-après, on décrira le chargement de différents produits en fonction de leurs caractéristiques, et notamment :

- un produit hydrophile de petite taille destiné à une administration systémique,
- d'un principe actif à activité anti-cancéreuse,
- de deux enzymes à activité antibactérienne, la lactopéroxydase et la glucose oxydase
- d'un extrait végétal composé d'oligomères procyanidoliques, possédant une activité anti-oxydante
- de constituants du globule gras du lait.

De même que les BVSM possédant une couronne d'acides gras greffés, les BVSM de type-L peuvent être stérilisés soit par filtration soit par autoclavage. Il peuvent également être congelés ou lyophilisés, par exemple en présence d'un additif ou encore atomisés.

Les avantages des BVSM de type-L ou de type-PS sont notamment :

- une construction modulaire autorisant l'adaptation au produit à vectoriser ou à présenter
- un biomimétisme vis-à-vis de structures naturelles comme les lipoprotéines ou les globules gras laitiers
- une plus grande facilité de synthèse et d'industrialisation par rapport aux BVSM comportant une couronne d'acide gras
- une grande stabilité chimique et thermique due à la structure polymérique du noyau
- une taille définie et la possibilité d'obtenir de façon homogène des très petites tailles (20 nm).
- leur capacité à stabiliser les composés encapsulés à l'intérieur du noyau et notamment des enzymes ou des composés antioxydants.

L'invention a donc pour objet une composition pharmaceutique, caractérisée en ce qu'elle contient un vecteur particulaire selon l'invention, et un support pharmaceutiquement acceptable pour son administration. Les vecteurs selon l'invention sont notamment utiles pour des applications thérapeutiques et en immunologie.

L'invention a également pour objet une composition cosmétologique, caractérisée en ce qu'elle contient un vecteur particulaire tels que décrits précédemment, et des excipients cosmétologiquement acceptables.

Enfin, les compositions alimentaires contenant des vecteurs particulaires selon l'invention font parties de l'invention.

Les exemples qui suivent sont destinés à illustrer l'invention sans en limiter la portée.

Dans ces exemples, on se réfèrera aux figures suivantes :

**Figure 1 :**   **transfert d'énergie sur des BVSM de type-L marqués par deux types de ligands fluorescents.** Le noyau polysaccharidique est marqué à l'aide de la Rhodamine et la couche phospholipidique est marquée à l'aide du NBD.

**Figure 2 :**   **transfert d'énergie entre des BVSM de type-PS marqués à l'aide de la Rhodamine et des liposomes marqués à l'aide du NBD.**

**Figure 3 :**   **Transfert d'énergie sur des BVSM marqués par deux types de ligands fluorescents.** Le coeur acylé est marqué à l'aide de la Rhodamine et la couche phospholipidique est marquée à l'aide du NBD.

**EXEMPLE 1: PRÉPARATION DE PARTICULES POLYSACCHARIDIQUES, DE DIAMETRE MOYEN DE 20 NANOMETRES, PAR DOUBLE RÉTICULATION DE DEXTRINE PAR L'OXYCHLORURE DE PHOSPHORE**

Dans un réacteur à double enveloppe de 3 litres on introduit 100g de dextrine (Roquette) que l'on dissout dans 350ml d'eau déminéralisée et 100ml de soude 10N.

Après homogénéisation, on rajoute simultanément 35,3ml de POCl3 et 225ml de soude 10N.

Après la fin de l'addition des réactifs, le mélange réactionnel est encore agité pendant 15 minutes puis neutralisé par addition d'acide chlorhydrique.

Le gel est dilué dans 2litres d'eau déminéralisée et homogénéisé à 900bars à l'aide d'un homogénéisateur à haute pression (Westfalia). Cette étape permet l'obtention de matrices de diamètre moyen de 20nm.

Les matrices sont ensuite lavées par précipitation à l'éthanol pour éliminer les sels puis séchées par lyophilisation à une concentration de 30g/l en matrices et de 20g/l en bicarbonate d'ammonium.

On récupère 75g de matrices lyophilisées (rendement de la réaction 75%)

**EXEMPLE 2: PREPARATION DE MATRICES POLYSACCHARIDIQUES GREFFEES PAR DES GROUPEMENTS CATIONIQUES AMMONIUMS QUATERNAIRES**

Dans un réacteur de 5 litres, on disperse 200 grammes d'amylopectine (Roquette, Lille, Fr.) dans 500 millilitres de soude 2N. Lorsque la solution est bien homogène, on introduit simultanément 93,6 grammes de glycidyltriméthylammonium chloride (Fluka, CH), correspondant à 0,5 équivalents /résidu glucose, solubilisé dans 150 millilitres d'eau, et 11,4 grammes (soit 9,7 millilitres) d'épichlorydrine (Fluka, CH) correspondant à 0,1 équivalents/résidu glucose. Le mélange est homogénéisé pendant 1 à 2 heures puis laissé au repos 8 heures. La préparation d'amidon polymérisé est ensuite amenée à pH 6 par addition d'acide acétique. Le gel obtenu est ensuite lavé plusieurs fois avec de l'eau distillée jusqu'à l'élimination de tous les sels et des sous-produits de la réaction.

On obtient, après lyophilisation, 244 grammes de gel réticulé, soit un rendement de la réaction de 80%.

**EXEMPLE 3: PREPARATION DE MATRICES POLYSACCHARIDIQUES GREFFEES PAR DES GROUPEMENTS ANIONIQUES DE TYPE CARBOXYMETHYL ("TYPE CM")**

Dans un réacteur de 5 litres, on solubilise 200 grammes de dextrine (Roquette) dans 300 millitres de soude 7 N. Lorsque la solution est bien homogène, on introduit simultanément 9,6 millilitres d'épichlorydrine (Fluka, CH) correspondant à 0,1 équivalents/résidu glucose et 117,2 grammes d'acide chloroacétique solubilisé dans 80 millilitres d'eau.

Après 1 heure d'agitation, on ajoute 9,6 millilitres d'épichlorhydrine et 150 millilitres de soude 20 N tout en agitant vigoureusement. Après la fin de l'addition, la préparation est homogénéisée 6 heures puis laissée au repos une nuit. Le gel ainsi obtenu est mis en suspension dans 1 litre d'eau et acidifié à pH 3-4 par ajout d'acide chlorhydrique 2 N. Le gel est ensuite filtré et lavé avec de l'eau distillée. On obtient après lyophilisation 276 grammes de gel de type Carboxyméthyl soit un rendement supérieur à 80%.

**EXEMPLE 4 : PRÉPARATION DE MATRICES HYDROPHILES, DE DIAMETRE MOYEN DE 1μM, PAR RÉTICULATION D'AMIDON PAR L'OXYCHLORURE DE PHOSPHORE**

Dans un réacteur à double enveloppe de 3 litres on introduit 100g d'amidon de blé (Roquette) que l'on dissout dans 375ml d'eau distillée et 100ml de soude 10N.

Le mélange est agité pendant 15 minutes à température ambiante.

Une fois le mélange homogénéisé on rajoute simultanément 11ml de $POCl_3$ et 50ml de soude 10N. Après la fin de l'addition des réactifs, le mélange réactionnel est encore agité, pendant 15 minutes, puis neutralisé à pH7 par addition d'acide acétique.

Le gel est lavé dans une essoreuse (Rousselet) pendant 30minutes avec de l'eau distillée de manière à éliminer les sels en excès et des sous produits de la réaction.

Le gel ainsi obtenu est ensuite homogénéisé à haute pression (500bars, homogénéisateur minilab Westfalia). Cette étape permet l'obtention de matrices d'une taille moyenne de 1μm. Le titrage de 1g de gel réticulé à l'aide d'un titrimètre automatique (titroprocesseur Methrom 682) révèle un taux de réticulation de 0,3meq de fonctions phosphodiester par gramme de gel réticulé.

On obtient ainsi des BVSM de type-PS de 1μm de diamètre.

**EXEMPLE 5 : OBTENTION DE PARTICULES POLYSACCHARIDIQUES IONIQUES DE 200 NANOMETRES**

15 grammes de gel obtenu selon l'exemple 2, ou de gel type CM obtenu selon l'exemple 3, sont dispersés dans 500 millilitres d'eau distillée et homogénéisés au moyen d'un homogénéisateur Rannie MiniLab 12-51 (APV Rannie, Copenhague, Dk). La pression d'homogénéisation appliquée est de 600 bars pendant 12 minutes.

On obtient une suspension fluide de particules de polysaccharides réticulés, basiques ou acides, dont la taille, mesurée par un Nanosizer Coulter N4MD, est centrée autour de 200 nanomètres. Les nanoparticules sont alors séchées par lyophilisation en présence de 20 grammes/litre de bicarbonate d'ammonium.

On obtient ainsi des BVSM de type-PS de 200nm de diamètre, utilisables tels quels ou transformés en BVSM de type-L.

**EXEMPLE 6 : PREPARATION DE BVSM DE TYPE L A PARTIR DE PARTICULES DE 20 NANOMETRES**

**Préparation de BVSM de type-L blancs**

10mg de noyaux polysaccharidiques 20nm sont dispersés dans 2ml d'Octyl Glucopyranoside OGP (Fluka) 50mM. On les mélange sous ultrasons à une solution de 10mg d'un mélange de lécithines de jaune d'oeuf purifiées (Sigma) et de Cholestérol (Sigma) 80/20 dispersés dans 2ml d'OGP 50mM. Cette préparation est ensuite diluée brutalement sous ultrasons jusqu'à une molarité finale en OGP de 5mM puis dialysée extensivement pendant 48 heures. L'analyse de la taille est effectuée à l'aide d'un nanosizer Coulter N4 indique que 97% des particules ont un diamètre de 23nm.

**EXEMPLE 7 : CARACTERISATION PAR TRANSFERT D'ENERGIE SUR BVSM DE TYPE L DE 20 NANOMETRES**

**A - Préparation des BVSM de type-L marqués par fluorescence**

**a - Préparation de noyaux polysaccharidiques marqués à la Rhodamine (λ ex540nm - λ ém580nm)**

50mg de noyaux polysaccharidiques 20nm, préparés selon l'exemple 6, sont dispersés dans 1ml de Bicarbonate de Sodium (Sigma) 100mM pH10. On rajoute 0,5mg d'isothiocyanate de Rhodamine B (Sigma) solubilisés dans du Diméthyl formamide (SDS), soit 1% de Rhodamine par rapport au poids de noyaux polysaccharidiques. Après 18 heures d'agitation à température ambiante plusieurs lavages à l'éthanol sont effectués afin d'éliminer la Rhodamine n'ayant pas réagi. Les noyaux sont ensuite séchés par lyophilisation.

**b - Etablissement du feuillet phospholipidique marqué au Nitrobenzodiazole (NBD) (λ ex470nm - λ ém530nm)**

**\* A partir de noyaux polysaccharidiques fluorescents**

10mg de noyaux marqués à l'aide de l'isothiocyanate de Rhodamine de 20nm sont dispersés en présence de 2ml d'Octyl Glucopyranoside OPG (Fluka) 50mM. On apporte ensuite à cette suspension 10mg d'un mélange de lécithines de jaune d'oeuf purifiées (Sigma), de Cholestérol (Sigma) et de phosphatidyléthanolamine marquée au Nitrobenzodiazole NBD (Sigma) 79/20/1 dispersés dans 2ml d'OGP 50mM, soit 100% de mélange phospholipidique par rapport aux noyaux polysaccharidiques. Cette solution est ensuite diluée brutalement jusqu'à une molarité finale en OGP de 5mM sous ultrasons puis dialysée extensivement pendant 48 heures.

**\* A partir de noyaux polysaccharidiques non fluorescents**

Les BVSM de type-L sont préparés de manière identique mais avec des noyaux polysaccharidiques non fluorescents. Cette préparation sert de témoin, elle représente le niveau zéro du transfert d'énergie.

**B - Préparation de noyaux polysaccharidiques fluorescents témoins : BVSM de type-PS**

10mg de noyaux fluorescents 20nm sont dispersés en présence de 2ml d'OGP 50mM. cette solution est ensuite diluée brutalement jusquà une molarité finale en OGP de 5mM sous ultrasons puis dialysée extensivement pendant 48 heures.

**C - Préparation de liposomes fluorescents témoins**

10mg du mélange phospholipidique précédent sont dispersés dans 2ml d'OGP 50mM. La solution est diluée brutalement jusquà une molarité finale en OGP de 5mM sous ultrasons puis dialysée extensivement pendant 48 heures.

**D - Préparation de BVSM témoins**

Des BVSM marqués par deux types de ligands fluorescents (coeur acylé marqué à l'aide de la Rhodamine et phospholipides marqués à l'aide du NBD) ainsi que des BVSM possédant des coeurs acylés non fluorescents et la couche phospholipidique marquée à l'aide du NBD ont été préparés selon les méthodes décrites dans les précédents brevets (EP 344 040).

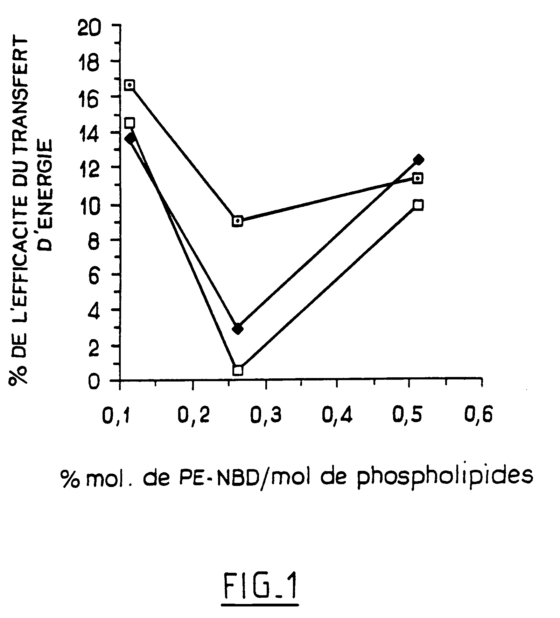

Le transfert d'énergie est quantifiée par la diminution de la fluorescence du NBD (longueur d'onde d'émission à 530nm).Un tranfert d'énergie de fluorescence de 20% a été mis en évidence sur les BVSM de type-L marqués par deux types de ligands fluorescents par rapport au BVSM de type-L ne possédant que la couche phospholipidique marquée en fluorescence (Fig. 1). Ce transfert reste stable après une incubation de 4 heures à 4° C et à 37° C ainsi qu'apès une incubation de 2 heures à 100° C. Ceci tend bien à démontrer que les phospholipides et les noyaux polysaccharidiques sont proches spatialement. L'association étroite phospholipides-noyaux polysaccharidiques est confirmée. Le simple mélange des noyaux polysaccharidiques fluorescents témoins (BVSM de type-PS) avec les liposomes fluorescents témoins n'entraine pas de transfert d'énergie entre les deux fluorophores, même dans les conditions d'incubation précédentes.

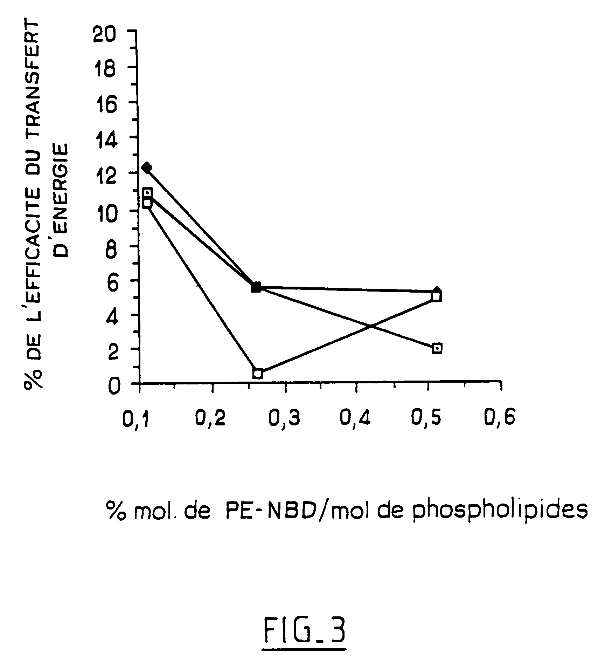

Le transfert d'énergie observé sur les BVSM de type-L est comparable avec les transferts d'énergie observés sur les BVSM dans les mêmes conditions expérimentales (Fig. 3).

**EXEMPLE 8 : PREPARATION DE BVSM DE TYPE L A PARTIR DE PARTICULES POLYSACCHARIDIQUES DE 200 NANOMETRES**

10 grammes de BVSM de type-PS préparés selon l'exemple 5 sont dispersés dans 500 millilitres d'eau distillée et placés dans un homogénéisateur Rannie Mini Lab 12-51 (APV Rannie, Copenhague), sous une pression de 300 bars, à un débit de 10 litres / heures. On injecte 0,5 grammes de phosphatidylcholine de soja hydrogénée (Nattermann, Fr) directement dans la suspension en recirculation dans l'homogénéisateur. On homogénéise cette préparation pendant 12 minutes.

La suspension obtenue contient des BVSM de type L d'environ 200 nm dispersés.

## EXEMPLE 9: PRÉPARATION D'UN SUCCÉDANÉ DE MATIERE GRASSE: ANCRAGE DE CONSTITUANTS PROTÉIQUES ET PHOSPHOLIPIDIQUES DU LAIT À LA SURFACE DE MATRICES HYDROPHILES PRÉPARÉES SELON L'EXEMPLE 4

100g de matrices hydrophiles préparées selon l'exemple 4 sont mis dans un mélangeur de 3 litres. 10g de constituants protéiques et phospholipidiques sont rajoutés.

On introduit, de manière progressive et sous agitation, 1 litre d'eau.

L'ensemble est homogénéisé à une vitesse de 80l/h et à 200bars (homogénéisateur Westfalia).

On obtient une préparation crémeuse ne contenant presque pas de constituants protéiques et phospholipidiques libres: deux témoins sont effectués avec des constituants protéiques et phospholipidiques seuls. Un témoin (n° 1) et la préparation sont centrifugés. On mesure le taux de protéines, par la méthode de Bradford, dans le témoin non centrifugé, dans le surnageant du témoin centrifugé et dans le surnageant de la préparation. Les analyses sont effectuées sur 100ml. On mesure 0,5g de protéines dans le surnageant du témoin centrifugé et 0,05g dans le surnageant de la préparation. On déduit de ces résultats, la quantité de protéines présente dans le centrifugeat du témoin n°1 soit 0,05g et, par la suite, la quantité de protéines ancrées sur les matrices hydrophiles soit 0,4g.

Le pourcentage de protéines ancrées est de 80% par rapport à la quantité de protéines introduites initialement.

D'autre part, le centrifugeat de la préparation est séché puis lavé, par filtration avec du chloroforme, de façon à extraire les phospholipides. Le chloroforme est ensuite évaporé et l'on pèse les phospholipides restants. La mesure donne 0,4g soit 80% par rapport à la quantité de constituants phospholipidiques introduits initialement.

## EXEMPLE 10 : INCORPORATION D'UNE ENZYME, LA GLUCOSE OXYDASE (GO) DANS LES BVSM TYPE-L

La glucose oxydase (GO) est une protéine acide ayant un point isoélectrique de 3,5 et un poids moléculaire moyen de 180 000 daltons.

On mélange, à l'état sec, 0,3 grammes de noyaux type QAE, obtenus selon l'exemple 2 puis 5, et 0,6 grammes de GO. Le mélange est ensuite progressivement hydraté par ajout de 1,5 millilitres de tampon à pH 7, au dessus du pI de la GO, sous agitation à température ambiante, puis laissé agité toute la nuit dans une chambre froide (4° C). Le pH est ensuite ajusté à 3, au dessous du pI de la GO et incubé pendant 30min supplémentaires. La préparation est alors dispersée dans 48,5 millilitres d'eau distillée, le pH ajusté à 7, et lyophilisé en présence de bicarbonate ammonium. Les noyaux chargés avec de la GO ainsi obtenus sont mélangés avec 0,15 grammes de phosphatidylcholine de soja hydrogénée (Nattermann, Fr.) en poudre. Le mélange est hydraté avec 150 millilitres d'eau distillée et homogénéisé dans un homogénéisateur Rannie Mini Lab 12-51 (APV Rannie, Copenhague, DK) sous une pression de 300 bars. On obtient ainsi des BVSM de type-L chargés avec de la GO, avec un rendement d'encapsulation de 92% et un taux d'incorporation de 184% par rapport au poids des noyaux, dosé par UV à 278 nm après ultrafiltration de la suspension.

## EXEMPLE 11 : INCORPORATION D'UNE ENZYME, LA LACTOPEROXYDASE (LP) DANS LES BVSM DE TYPE-PS

La lactoperoxidase (LP) est une enzyme antibactérienne. C'est une protéine basique possédant un point isoélectrique de 9,6 et un poids moléculaire moyen d'environ 80 000 daltons.

On met en suspension 0,5 grammes de BVSM type-PS anioniques (CM), obtenu selon l'exemple 3 puis 5, dans 100 millilitres d'un tampon ajusté à pH 7, en dessous du pI de la LP, dans un ballon de 250 ml. On introduit ensuite 0,5 g de LP (BioSerae) solubilisée dans 1 millilitre d'eau, sous agitation.

Le mélange est agité pendant une nuit dans une chambre froide (4° C). Le pH est ensuite ajusté à 9,8, au -dessus du pI de la LP, et incubé pendant 30min. Le pH est alors ramené à 7 puis la suspension est lyophilisée en présence de bicarbonate d'ammonium (20 grammes/litre). On obtient des BVSM de type-PS chargés en LP avec un rendement de chargement de 99% et un taux d'incorporation de 99% par rapport au poids des noyaux d'après le dosage par UV à 412 nm.

## EXEMPLE 12: ACTIVITÉ ANTI-BACTÉRIENNE DU MÉLANGE LP ENCAPSULÉE DANS DES BVSM DE TYPE-PS ET GO ENCAPSULÉE DANS DES BVSM DE TYPE-L

### * Dosage de l'activité in vitro:

On mélange 1 ml d'une solution de glucose 1M, 0,2 ml d'une solution d'ortho-phénylène diamine (1 mg/ml dans un tampon citrate pH 5,6 concentration 10mM) et 1,6 ml d'un solution tampon citrate (10 mM, pH 5,6).

On mélange par ailleurs 0,2 mg de BVSM type-PS contenant de la Lactopéroxydase, préparés suivant l'exemple 11 et 1,8 mg de BVSM type-L contenant de la Glucose Oxydase, préparés suivant l'exemple 10 dans 1 ml d'eau.

On ajoute 0,2 ml de cette suspension aux 2,8 ml préparés plus haut et on agite 2 minutes à température ambiante.

La réaction colorée qui se développe est arrêtée par 1 ml d'acide sulfurique 2N et la coloration est lue à 490 nm contre un blanc ne contenant pas de BVSM.

La présence d'une coloration intense montre que les deux enzymes encapsulées dans les BVSM de type-L conservent leur activité. Le dosage de l'activité des enzymes encapsulées donne un résultat comparable à celui obtenu avec les enzymes en solution aqueuse à la même concentration.

**\* Activité anti-bactérienne contre une souche d'Escherichia coli:**

On prépare un milieu de culture LB Glucose mélangé à une gélose Agar, coulé dans des boîtes à antibiogramme. La souche d'E. coli est ensemencée à la surface de la gélose à raison de 200 µl / boîte. Des disques papier stériles sont imprégnés de suspensions d'enzymes, encapsulées ou non et déposés sur la gélose des boites ensemencées. On laisse incuber 24h à 37°C et on mesure les diamètres d'inhibition autour des disques.

Les disques ont été imprégnés avec des concentrations en enzymes variant de 0,05 à 0,6 mg/ml de LP et de 0,8 à 10 mg/ml de GO. Les diamètres d'inhibition varient de 12 à 20 mm et sont comparables que les enzymes aient été encapsulées ou non.

## EXEMPLE 13: STABILISATION DE L'ACTIVITÉ ANTIBACTÉRIENNE DES ENZYMES ENCAPSULÉES DANS LES BVSM

Trois lots de glucose oxydase ont été préparés. Le lot 1 est constitué d'une solution aqueuse de GO à 0,2 mg/ml, le lot 2 par une suspension aqueuse de GO encapsulée dans des BVSM préparés selon les précédents brevets, avec un taux d'encapsulation de 200% par rapport au poids de particules et dont la concentration en GO est de 0,2 mg /ml et le lot 3 par une suspension aqueuse de GO encapsulée dans des BVSM de type-L selon l'exemple 10, et dont la concentration en GO est également de 0,2 mg/ml.

Ces trois suspensions sont laissées à 4° C et dosées toutes les semaines puis tous les mois par la méthode décrite à l'exemple 12. L'activité de la solution de GO décroît au cours du temps et devient nulle au bout de 250 jours. A l'inverse, l'activité des deux suspensions de GO encapsulée soit dans les BVSM soit dans les BVSM de type-L reste constante et demeure égale à 100% de l'activité initiale au bout de 320 jours.

Par ailleurs, sont également préparés une solution aqueuse de lactopéroxydase à 0,1 mg/ml et une suspension de LP encapsulées dans des BVSM de type-PS préparés selon l'exemple 11 et dont la concentration en LP est également de 0,1 mg/ml.

Ces deux suspensions sont laissées à 4° C et dosées toutes les semaines puis tous les mois par la méthode décrite à l'exemple 12. L'activité de la solution de LP décroît au cours du temps et, au bout de 90 jours, l'activité résiduelle n'est plus que de 35% de celle de l'acticité initiale. A l'inverse, l'activité de la suspension de LP encapsulée dans les BVSM de type-PS reste constante et demeure égale à 100% de l'activité initiale au bout de 90 jours.

## EXEMPLE 14 : INCORPORATION D'OLIGOMERES PROCYANIDOLIQUES (OPC) DANS LES BVSM DE TYPE-L OU COMPLETS

### A - Incorporation d'OPC dans un gel de type ammonium quaternaire

Les oligomères procyanidoliques (OPC) sont des agents antioxydants de la famille des tannins hydrolysables (flavonoïdes). Ils présentent des propriétés anti-radicaux libres. Leur poids moléculaire moyen est d'environ 2 000 daltons. Ils sont solubles dans l'eau à un pH inférieur à 7.

On solubilise 5 grammes d'OPC dans une quantité minimum d'eau distillée (environ 5 millilitres) et on introduit 10 grammes de gel type ammonium quaternaire, préparé selon l'exemple 2. Le mélange est hydraté avec 180 millilitres de tampon à pH 6, au dessus du pKa moyen des OPC, sous agitation pour obtenir une pâte homogène. Le mélange est laissé agité une nuit. Le pH est alors ramené à 3,5, au dessous du pKa moyen des OPC, laissé incubé 30 min. sous agitation puis lavé à l'eau distillée par centrifugation afin d'éliminer les OPC libres. La quantité d'OPC libre dans le surnageant est dosée par UV à 280 nm. On obtient un rendement d'encapsulation de 82% et un taux de chargement de 41% par rapport au poids du gel.

Ce gel chargé est ensuite dispersé dans 500 millilitres d'eau distillée et homogénéisé dans un homogénéisateur Rannie Mini Lab 12-51 (APV Rannie, Copenhague, DK) sous une pression de 600 bars pendant 15 minutes à un débit de 10 litres/heures. On obtient une suspension de BVSM de type PS chargées avec des OPC à une taille centrée autour de 200 nm.

### B - Obtention de BVSM de type L de 200 nm chargés en OPC

La suspension de BVSM de type PS obtenue précédemment est placée dans un homogénéisateur Rannie Mini Lab 12-51, sous une pression de 300 bars.

On injecte alors 0,5 grammes de phosphatidylcholine de soja hydrogénée (Nattermann, Fr.) directement dans la suspension en recirculation dans l'homogénéisateur, sous une pression de 300 bars et on homogénéise pendant 12 minutes avec un débit de 10 litres/heure. La supension obtenue contient des particules d'environ 200 nm dispersées.

**C - Obtention de BVSM "complets" de 200 nm chargés en OPC :**

Des BVSM "complets" sont préparés tel que précédemment décrit au moyen des BVSM de type PS chargés en OPC par séchage (lyophilisation), acylation régiosélective dans un solvant organique et établissement d'un feuillet externe de phospholipides.

**D - Stabilisation d'oligomères procyanidoliques (OPC) des les BVSM de type L**

Une émulsion de type huile dans eau est préparée et divisée en trois lots. Des OPC sont incorporés dans le premier lot à la concentration finale de 0,05 %. Dans un deuxième lot sont incorporés des OPC encapsulés dans des BVSM de type L et dans le troisième lot sont incorporés des OPC encapsulés dans des BVSM "complets". La concentration finale de ces deux derniers lots est également de 0,05 % en OPC; Ces trois émulsions présentent une couleur blanc crème homogène.

Un échantillon de chacun des trois lots est placé sous une lampe UV (254 nm) pendant 48 heures puis laissé à l'abri de la lumière. Après 10 jours, l'émulsion contenant les OPC libres présente une coloration brune très intense, l'émulsion contenant des OPC encapsulés dans des BVSM de type L présente une coloration blanc crème légèrement plus soutenue alors que l'émulsion contenant des OPC encapsulés dans des BVSM "complets" n'a pas évolué.

De la même façon, la stabilité des OPC dans ces émulsions est testée pendant 6 mois à 40°C. La crème contenant les OPC encapsulés dans des BVSM "complets" n'a pas changé significativement de coloration, celle contenant les OPC encapsulés dans des BVSM de type L a une couleur légèrement plus intense alors que celle contenant des OPC libres est devenue brunâtre.

**EXEMPLE 15 : INCORPORATION D'UN ANTIBIOTIQUE ANTICANCÉREUX, LA DOXORUBICINE, DANS DES BVSM DE TYPE-L EN UTILISANT LE MODE DE CHARGEMENT PAR CONTROLE TOPOLOGIQUE**

La doxorubicine est un antibiotique anticancéreux appartenant à la famille des anthracyclines. C'est un produit amphiphile caractérisé par un aglycone polyaromatique conférant à la molécule des propriétés de fluorescence caractéristiques et par un sucre aminé, la daunosamine. Le poids moléculaire du chlorhydrate est de 580 et son pKa est de 8,5.

On utilise des noyaux polysaccharidiques préparés comme précédemment.

**1- Incorporation de la doxorubicine sans contrôle topologique**

La doxorubicine (0,1g) en solution aqueuse est ajoutée progressivement aux noyaux polysaccharidiques (0,5g) sous agitation magnétique. La suspension obtenue est ensuite laissée sous agitation pendant 17h à température ambiante et à l'abri de la lumière.

Les noyaux polysaccharidiques ainsi chargés en doxorubicine ont complètement précipité. Même en présence de détergent et de phospholipides, ils ne peuvent pas être dispersés correctement avec une taille de 20nm.

L'incorporation de la doxorubicine sans contrôle topologique conduit à l'agrégation totale des noyaux polysaccharidiques et ne peut être utilisée pour former des BVSM de type-L de 20nm.

**2- Incorporation de la doxorubicine avec contrôle topologique**

La doxorubicine (0,1g) en solution aqueuse est ajoutée progressivement aux noyaux polysaccharidiques (0,5g) sous agitation magnétique. Le pH est ajusté à 7, en-dessous du pKa de la doxorubicine, pendant l'ajout. La suspension obtenue est agitée pendant 17h à température ambiante et à l'abri de la lumière. Le pH est ensuite ajusté à 9, au-dessus de pKa de la doxorubicine, et incubé pendant 30min. La suspension de noyaux polysaccharidiques est ensuite divisée en deux : une partie est analysée en tant que noyaux polysaccharidiques, l'autre partie des noyaux polysaccharidiques est préparée en BVSM de type L.

**a- BVSM de type PS**

Après l'étape d'incubation à pH 9, la suspension obtenue est diluée dans 1l d'eau et ramené à pH 7. Les noyaux ainsi chargés sont analysés : la filtration sur 0,2μm de la suspension présente un rendement supérieur à 95% en doxorubicine, ce qui indique que la taille des noyaux polysaccharidiques est de 20nm, et l'ultrafiltration centrifuge d'un aliquot de la suspension met en évidence l'absence de doxorubicine libre. Les résultats indiquent la présence de 4mg de doxorubicine dans l'ultrafiltrat et de 46mg de doxorubicine dans les noyaux polysaccharidiques, ce qui correspond à un rendement de 92% et un taux d'encapsulation de 18% en doxorubicine. La filtration sur 0,2μm de la suspension obtenue présente un rendement supérieur à 95%, ce qui indique que la taille des BVSM est de 20nm.

**b- BVSM de type L**

Les noyaux polysaccharidiques sont ensuite dispersés avec les phospholipides (0,25g) (EYPC / cholestérol 80/20 en poids) prédispersés dans de l'hécameg 50mM à une concentration de 10g/l. La suspension obtenue est diluée brutalement dans 1l d'eau. Le détergent est ensuite éliminé par ultrafiltration. La doxorubicine est ensuite dosée dans l'ultrafiltrat et dans les BVSM obtenus. Les résultats indiquent la présence de 2,5 mg de doxorubicine dans l'ultrafiltrat et de 47,5 mg de doxorubicine dans les BVSM, ce qui correspond à un rendement en doxorubicine de 95% et un taux d'encapsulation en doxorubicine de 19% par rapport aux noyaux polysaccharidiques. La filtration sur 0,2μm de la suspension obtenue présente un rendement supérieur à 95%, ce qui indique que la taille des BVSM est de 20nm.

**EXEMPLE 16 : POST-CHARGEMENT DE LA DOXORUBICINE DANS DES NOYAUX POLYSACCHARIDIQUES**

La doxorubicine est un antibiotique anticancéreux appartenant à la famille des anthracyclines. C'est un produit amphiphile caractérisé par un aglycone polyaromatique, conférant à la molécule des propriétés de fluorescence caractéristiques, et par un sucre aminé, la daunosamine. Le poids moléculaire du chlorhydrate est de 580 et son pKa est de 8,2-8,5.

**\* Incorporation de la doxorubicine dans les noyaux polysaccharidiques**

Les noyaux polysaccharidiques utilisés ont été réticulés et fonctionnalisés par $POCl_3$ et possèdent une taille 20 nm. Leur densité ionique est de 1,59mequivPO4/g..

Les noyaux polysaccharidiques (10mg) sont dispersés dans l'eau (10ml) sous ultra-sons. Le pH de la suspension de noyaux est ajusté à 7 avec NaOH 0,1N. La doxorubicine (4,6mg) en solution dans l'eau à 5mg/ml est ajoutée lentement sous sonication par 20μl. Le pH est ajusté à 7 si nécessaire avec NaOH 0,1N.

Les noyaux polysaccharidiques ainsi chargés sont caractérisés par :

- leur aptitude à filtrer sur 0,2μm : le rendement de filtration est déterminé par le rapport des concentrations avant et après filtration.
  Après incorporation, 100μl de la suspension de noyaux polysaccharidiques chargés sont prélevés pour déterminer la concentration en doxorubicine. Le reste de la suspension est filtré sur membrane de porosité 0,2μm. Un aliquot de 100μl est à nouveau prélevé pour le dosage de la doxorubicine. La doxorubicine est dosée par CLHP après libération des noyaux polysaccharidiques.

$$\text{Rendement de filtration sur } 0,2\mu m \ (\%) = \frac{\text{concentration en doxorubicine après filtration}}{\text{concentration en doxorubicine avant filtration}} \times 100$$

- la fraction non-incorporée qui est déterminée par ultrafiltration centrifuge. Après filtration, 1ml de la suspension de noyaux polysaccharidiques dilués au 1/2 est déposé sur le système d'ultrafiltration centrifuge (Microsep) puis centrifugé à 7500g pendant 30 min. L'ultrafiltrat obtenu est dosé par CLHP en doxorubicine.

$$\text{Fraction non-incorporée } (\%) = \frac{\text{concentration en doxorubicine dans l'ultrafiltrat}}{\text{concentration en doxorubicine après filtration}} \times 100$$

Le rendement de filtration est de 97 % et la fraction non-incorporée est inférieure à 5%, conduisant à un rendement d'incorporation de 99 %.

**\* Comparaison du comportement en conditions physiologiques de la doxorubicine incorporée dans les noyaux polysaccharidiques et dans les BVSM**

Les particules post-chargées en doxorubicine, noyaux polysaccharidiques ou BVSM, sont incubées en PBS à 37°C à une concentration en doxorubicine de 50μg/ml au temps 0h et 4h, 1ml de la suspension de particules est prélevé et ultrafiltré par centrifugation (7500g, 30min) sur Microsep pour déterminer la fraction de doxorubicine libérée.

L'ultrafiltrat obtenu est ensuite dosé en doxorubicine par CLPH. Les résultats sont présentés dans le tableau suivant :

| % de doxorubicine restant incorporée | Type de particules NPS | Type de particules BVSM |
|---|---|---|
| temps 0h | 67+/-1 | 62+/-1 |
| temps 4h | 64+/-3 | 55+/-5 |

## Comportement en conditions physiologiques des noyaux polysaccharidiques et des BVSM, incorporés en doxorubicine par post-chargement

Les résultats obtenus indiquent une différence de comportement de la doxorubicine incorporée dans ces deux types de particules : la doxorubicine reste incorporée de manière plus forte dans les noyaux polysaccharidiques que dans les BVSM.

**EXEMPLE 17 : POST-CHARGEMENT DE LA DOXORUBICINE DANS LES BVSM**

**\* Incorporation de la doxorubicine dans les BVSM (de type L ou non)**

Les BVSM sont préparés à partir de noyaux acylés sur lesquels le feuillet phospholipidique (100 % en poids) est établi par la méthode de détergent.

Brièvement, les phospholipides (10mg) de composition EYPC / cholestérol 80/20 (en poids) sont dispersés dans de l'Hecameg 50nM (2ml). Les noyaux acylés (10mg) sont ajoutés aux phospholipides puis soumis au bain à ultra-sons pendant 5min. La suspension obtenue est diluée dans 8ml d'eau distillée sous la CMC de l'Hecameg qui est de 20nM puis dialysée extensivement. Les BVSM obtenus sont filtrés sur 0,2μm et conservés stérilement.

Les BVSM de type L sont préparés selon le même protocole mais en utilisant des noyaux polysaccharidiques à la place des noyaux acylés.

Le pH de la suspension de BVSM (20mg) est ajusté à 7 avec NaOH 0,1N. La doxorubicine (4,6 mg) en solution dans l'eau à 5mg/ml est ajoutée lentement sous sonication par 20μl. Le pH est ajusté à 7 si nécessaire avec NaOH 0,1N.

Les BVSM ainsi chargés sont caractérisés par :

- leur aptitude à filtrer sur 0,2μm : le rendement de filtration est déterminé par le rapport des concentrations avant et après filtration.

  Après incorporation, 100μl de la suspension de BVSM chargés sont prélevés pour déterminer la concentration en doxorubicine. Le reste de la suspension est filtré sur membrane de porosité 0,2μm. Un aliquot de 100μl est à nouveau prélevé pour le dosage de la doxorubicine. La doxorubicine est dosée par CLHP après libération des BVSM;

$$\text{Rendement de filtration sur } 0,2\mu m \, (\%) = \frac{\text{concentration en doxorubicine après filtration}}{\text{concentration en doxorubicine avant filtration}} \times 100$$

- la fraction non-incorporée qui est déterminée par ultrafiltration centrifuge. Après filtration, 1ml de la suspension de BVSM dilués au 1/2 est déposé sur le système d'ultrafiltration centrifuge (Microsep) puis centrifugé à 7500g pendant 30min. L'ultrafiltrat obtenu est dosé par CLHP en doxorubicine.

$$\text{Fraction non-incorporée } (\%) = \frac{\text{concentration en doxorubicine dans l'ultrafiltrat}}{\text{concentration en doxorubicine après filtration}} \times 100$$

Le rendement de filtration est de 95 % et la fraction non-incorporée est inférieure à 5% conduisant à un rendement d'incorporation de 99 %.

**\* Comportement en conditions physiologiques de la doxorubicine incorporée dans les BVSM de type L**

Les particules post-chargées en doxorubicine, BVSM de type L ou non, sont incubées en PBS à 37°C à une concentration en doxorubicine de 50µg/ml au temps 0h et 4h, 1ml de la suspension de particules est prélevé et ultrafiltré par centrifugation (7500g, 30 min) sur Microsep pour déterminer la fraction de doxorubicine libérée. L'ultrafiltrat obtenu est ensuite dosé en doxorubicine par CLHP.
Les résultats sont présentés dans le tableau suivant :

| % de doxorubicine restant incorporée | BVSM de type L | BVSM |
|---|---|---|
| temps 0h | 58+/-1 | 62+/-1 |
| temps 4h | 54+/-3 | 55+/-5 |

Comportement en conditions physiologiques des BVSM, de type L ou non, incorporés en doxorubicine par post-chargement

Les résultats obtenus indiquent que les BVSM de type L, chargés en doxorubicine, présentent un comportement en conditions physiologiques similaire à celui des BVSM.

Légende de la FIG. 1 :

▣ 4°C

◆ 37°C

☐ 100°C

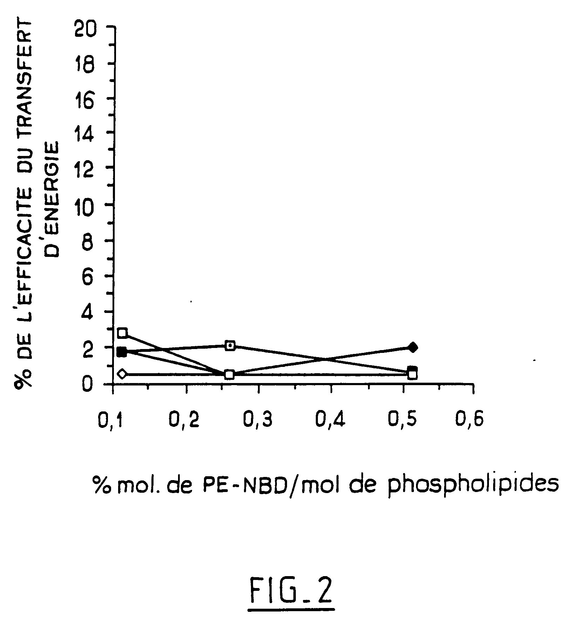

Légende de la FIG. 2 :

▣ 4°C

◆ 37°C

☐ 100°C

◇ 37°C Sonique

Légende de la FIG. 3 :

▣ 4°C

◆ 37°C

☐ 100°C

EP 0 687 173 B1

## Revendications

1. Vecteur particulaire synthétique, caractérisé en ce qu'il comporte :

   - un noyau hydrophile non liquide,
   - une couche externe constituée au moins en partie de composés amphiphiles, associée au noyau par des interactions hydrophobes et/ou des liaisons ioniques.

2. Vecteur particulaire selon la revendication 1, caractérisé en ce que le noyau hydrophile est constitué d'une matrice de polysaccharides ou d'oligosaccharides naturellement ou chimiquement réticulés.

3. Vecteur particulaire selon l'une des revendications 1 et 2, caractérisé en ce que des ligands ioniques sont greffés sur le noyau hydrophile.

4. Vecteur particulaire selon l'une des revendications 1 à 3, caractérisé en ce que les composés amphiphiles de la couche externe sont choisis dans le groupe comprenant les phospholipides, les céramides, les tensioactifs ioniques, les tensioactifs non ioniques.

5. Vecteur particulaire selon l'une des revendications 1 à 4, caractérisé en ce que la couche externe comprend en outre au moins un composé lipidique choisi dans le groupe comprenant le cholestérol, les acides gras naturels et les vitamines liposolubles.

6. Vecteur particulaire selon l'une des revendications 1 à 5, caractérisé en ce qu'il comporte en outre un principe actif, situé dans le noyau ou dans la couche externe.

7. Vecteur particulaire selon l'une des revendications 1 à 6, caractérisé en ce qu'il a un diamètre compris entre 10nm et 5µm.

8. Vecteur particulaire selon l'une des revendications 1 à 7 caractérisé en qu'il a un diamètre compris entre 20 et 200 nm.

9. Vecteur particulaire selon l'une des revendications 1 à 8, caractérisé en ce qu'il contient un principe actif choisi dans le groupe comprenant :

   - les antibiotiques et les anti-viraux,
   - les protéines, les protéoglycanes, les peptides,
   - les polysaccharides, les lipopolysaccharides,
   - les anticorps,
   - les antigènes,
   - les insecticides et fongicides,
   - les composés agissant sur le système cardio-vasculaire,
   - les anti-cancéreux,
   - les anti-malariques,
   - les anti-asthmatiques,
   - les composés présentant une action sur la peau,
   - les constituants des globules gras laitiers.

10. Procédé de préparation d'un vecteur particulaire selon l'une des revendications 1 à 9, caractérisé en ce que :

    a) on effectue l'encapsulation d'un principe actif ionisable acide ou basique, dans une matrice hydrophile réticulée greffée par des ligands d'une espèce ionique complémentaire de celle du principe actif, à un pH auquel le principe actif se trouve sous forme ionisée,
    b) on fait varier le pH du milieu, par rapport au $pK_a$ du principe actif, jusqu'à une valeur à laquelle le principe actif n'est pas sous forme ionisée,
    c) on récupère la matrice hydrophile comportant le principe actif localisé principalement au centre de ladite matrice.

11. Procédé de préparation d'un vecteur particulaire, selon la revendication 10, caractérisé en ce que :

a) on effectue l'encapsulation d'au moins un principe actif ionisable basique, dans une matrice hydrophile réticulée greffée par des ligands ioniques acides, à un pH inférieur au $pK_a$ du principe actif,

b) on augmente le pH du milieu jusqu'à une valeur supérieure au $pK_a$ du principe actif,

c) on récupère la matrice hydrophile comportant le principe actif localisé principalement au centre de ladite matrice.

**12.** Procédé de préparation d'un vecteur particulaire, selon la revendication 10, caractérisé en ce que :

a) on effectue l'encapsulation d'au moins un principe actif ionisable acide, dans une matrice hydrophile réticulée greffée par des ligands ioniques basiques, à un pH supérieur au $pK_a$ du principe actif,

b) on diminue le pH du milieu jusqu'à une valeur inférieure au $pK_a$ du principe actif,

c) on récupère la matrice hydrophile comportant le principe actif localisé principalement au centre de ladite matrice.

**13.** Procédé selon l'une des revendications 10 à 12, caractérisé en ce que la matrice hydrophile est constituée de polysaccharides ou d'oligosaccharides, naturellement ou chimiquement réticulés.

**14.** Procédé selon l'une des revendications 10 à 13, caractérisé en ce que l'on fixe une couche de composés amphiphiles sur la matrice obtenue à l'issue de l'étape c).

**15.** Vecteur particulaire tel qu'il peut être obtenu par le procédé selon l'une des revendications 10 à 14.

**16.** Composition pharmaceutique, caractérisée en ce qu'elle contient un vecteur particulaire selon l'une des revendications 1 à 9 ou obtenu par un procédé selon l'une des revendications 10 à 14, et un support pharmaceutiquement acceptable pour son administration.

**17.** Composition cosmétologique, caractérisée en ce qu'elle contient un vecteur particulaire selon l'une des revendications 1 à 9 ou susceptible d'être obtenu par un procédé selon l'une des revendications 10 à 14, et des excipients cosmétologiquement acceptables.

**18.** Composition à usage alimentaire, caractérisée en ce qu'elle contient un vecteur selon l'une des revendications 1 à 9 ou susceptible d'être obtenu par un procédé selon l'une des revendications 10 à 14.

**19.** Utilisation pour le production l'un médicament d'un vecteur selon l'une des revendications 1 à 9 ou susceptible d'être obtenu par un procédé selon l'une des revendications 10 à 14.

## Claims

**1.** Synthetic particulate vector, characterized in that it comprises:

- a non-liquid hydrophilic core,
- an external layer composed at least in part of amphiphilic compounds, combined with the core via hydrophobic interactions and/or ionic bonds.

**2.** Particulate vector according to Claim 1, characterized in that the hydrophilic core is composed of a matrix of polysaccharides or of oligosaccharides which are naturally or chemically crosslinked.

**3.** Particulate vector according to either of Claims 1 and 2, characterized in that ionic ligands are grafted onto the hydrophilic core.

**4.** Particulate vector according to one of Claims 1 to 3, characterized in that the amphiphilic compounds of the external layer are chosen from the group comprising phospholipids, ceramides, ionic surface-active agents or nonionic surface-active agents.

**5.** Particulate vector according to one of Claims 1 to 4, characterized in that the external layer additionally comprises at least one lipid compound chosen from the group comprising cholesterol, natural fatty acids and fat-soluble vitamins.

**6.** Particulate vector according to one of Claims 1 to 5, characterized in that it additionally contains an active principle situated in the core or in the external layer.

7. Particulate vector according to one of Claims 1 to 6, characterized in that it has a diameter of between 10 nm and 5 μm.

8. Particulate vector according to one of Claims 1 to 7, characterized in that it has a diameter of between 20 and 200 nm.

9. Particulate vector according to one of Claims 1 to 8, characterized in that it contains an active principle chosen from the group comprising:

   - antibiotics and antivirals,
   - proteins, proteoglycans, peptides,
   - polysaccharides, lipopolysaccharides,
   - antibodies,
   - antigens,
   - insecticides and fungicides,
   - compounds which act on the cardiovascular system,
   - anticancers,
   - antimalarials,
   - antiasthmatics,
   - compounds having an effect on the skin,
   - constituents of dairy fat globules.

10. Process for the preparation of a particulate vector according to one of Claims 1 to 9, characterized in that:

    a) an acidic or basic ionizable active principle is encapsulated in a crosslinked hydrophilic matrix grafted by ligands of an ionic species complementary with that of the active principle, at a pH at which the active principle is in the ionized form, b) the pH of the medium is varied, with respect to the
    $pK_a$ of the active principle, to a value at which the active principle is not in the ionized form,
    c) the hydrophilic matrix, containing the active principle localized mainly at the centre of the said matrix, is recovered.

11. Process for the preparation of a particulate vector according to Claim 10, characterized in that:

    a) at least one basic ionizable active principle is encapsulated in a crosslinked hydrophilic matrix grafted by acidic ionic ligands, at a pH below the $pK_a$ of the active principle,
    b) the pH of the medium is increased to a value above the $pK_a$ of the active principle,
    c) the hydrophilic matrix, containing the active principle localized mainly at the centre of the said matrix, is recovered.

12. Process for the preparation of a particulate vector according to Claim 10, characterized in that:

    a) at least one acidic ionizable active principle is encapsulated in a crosslinked hydrophilic matrix grafted by basic ionic ligands, at a pH above the $pK_a$ of the active principle,
    b) the pH of the medium is decreased to a value below the $pK_a$ of the active principle,
    c) the hydrophilic matrix, containing the active principle localized mainly at the centre of the said matrix, is recovered.

13. Process according to one of Claims 10 to 12, characterized in that the hydrophilic matrix is composed of polysaccharides or of oligosaccharides which are naturally or chemically crosslinked.

14. Process according to one of Claims 10 to 13, characterized in that a layer of amphiphilic compounds is fixed to the matrix obtained on completion of the stage c).

15. Particulate vector, such that it can be obtained by the process according to one of Claims 10 to 14.

16. Pharmaceutical composition, characterized in that it contains a particulate vector according to one of Claims 1 to 9 or obtained by a process according to one of Claims 10 to 14 and a pharmaceutically acceptable support for its administration.

17. Cosmetological composition, characterized in that it contains a particulate vector according to one of Claims 1 to 9 or capable of being obtained by a process according to one of Claims 10 to 14 and cosmetologically acceptable excipients.

18. Composition for food use, characterized in that it contains a vector according to one of Claims 1 to 9 or capbale of being obtained by a process according to one of Claims 10 to 14.

19. Use, for the production of a medicament, of a vector according to one of Claims 1 to 9 or capbale of being obtained by a process according to one of Claims 10 to 14.

**Patentansprüche**

1. Synthetischer partikulärer Vektor, dadurch gekennzeichnet, daß er umfaßt:

   - einen nicht-flüssigen hydrophilen Kern,
   - eine äußere Schicht, die mindestens zum Teil aus amphiphilen Verbindungen besteht, die durch hydrophobe Wechselwirkungen und/oder ionische Bindungen mit dem Kern verbunden (assoziiert) ist.

2. Partikulärer Vektor nach Anspruch 1, dadurch gekennzeichnet, daß der hydrophile Kern aus einer Matrix aus Polysacchariden oder Oligosacchariden besteht, die in natürlicher oder chemischer Weise vernetzt worden sind.

3. Partikulärer Vektor nach einem der Ansprüche 1 und 2, dadurch gekennzeichnet, daß ionische Liganden auf den hydrophilen Kern aufgepfropft sind.

4. Partikulärer Vektor nach einem der Ansprüche 1 bis 3, dadurch gekennzeichnet, daß die amphiphilen Verbindungen der äußeren Schicht ausgewählt werden aus der Gruppe, die umfaßt die Phospholipide, die Ceramide, die ionischen Tenside und die nicht-ionischen Tenside.

5. Partikulärer Vektor nach einem der Ansprüche 1 bis 4, dadurch gekennzeichnet, daß die äußere Schicht außerdem mindestens eine Lipid-Verbindung umfaßt, die ausgewählt wird aus der Gruppe, die umfaßt das Cholesterin, die natürlichen Fettsäuren und die fettlöslischen Vitamine.

6. Partikulärer Vektor nach einem der Ansprüche 1 bis 5, dadurch gekennzeichnet, daß sie außerdem einen Wirkstoff umfaßt, der im Kern oder in der äußeren Schicht angeordnet ist.

7. Partikulärer Vektor nach einem der Ansprüche 1 bis 6, dadurch gekennzeichnet, daß er einen Durchmesser zwischen 10 nm und 5 $\mu$m aufweist.

8. Partikulärer Vektor nach einem der Ansprüche 1 bis 7, dadurch gekennzeichnet, daß er einen Durchmessern zwischen 20 und 200 nm aufweist.

9. Partikulärer Vektor nach einem der Ansprüche 1 bis 8, dadurch gekennzeichnet, daß er einen Wirkstoff enthält, der ausgewählt wird aus der Gruppe, die umfaßt:

   - die Antibiotika und die Antivira,
   - die Proteine, die Proteoglycane, die Peptide,
   - die Polysaccharide, die Lipopolysaccharide,
   - die Antikörper,
   - die Antigene,
   - die Isektizide und die Fungizide,
   - die Verbindungen, die auf das cardiovasculäre System einwirken,
   - die Antikrebsmittel,
   - die Antimalariamittel,
   - die Antiasthmamittel,
   - die Verbindungen, die eine Wirkung auf die Haut ausüben, und
   - die Bestandteile der Milchfettkörper (-kügelchen).

10. Verfahren zur Herstellung eines partikulären Vektors nach einem der Ansprüche 1 bis 9, dadurch gekennzeichnet, daß man

a) einen sauren oder basischen ionisierbaren Wirkstoff in eine vernetzte hydrophile Matrix, die mit Liganden einer Ionenart bepfropft ist, die komplementär zu derjenigen des Wirkstoffes ist, bei einem pH-Wert, bei dem der Wirkstoff in ionisierter Form vorliegt, einkapselt,

b) den pH-Wert des Mediums gegenüber dem pKa-Wert des Wirkstoffes variieren läßt bis zu einem Wert, bei dem der Wirkstoff nicht mehr in ionisierter Form vorliegt, und

c) die hydrophile Matrix, die den hauptsächlich im Zentrum der genannten Matrix lokalisierten Wirkstoff enthält, abtrennt (zurückgewinnt).

11. Verfahren zur Herstellung eines partikulären Vektors nach Anspruch 10, dadurch gekennzeichnet, daß man

a) mindestens einen basischen ionisierbaren Wirkstoff in eine vernetzte hydrophile Matrix, die mit sauren ionischen Liganden bepfropft ist, bei einem pH-Wert unterhalb des pKa-Wertes des Wirkstoffes einkapselt,

b) den pH-Wert des Mediums bis auf einen Wert oberhalb des pKa-Wertes des Wirkstoffes erhöht und

c) die hydrophile Matrix, die den hauptsächlich im Zentrum der genannten Matrix lokalisierten Wirkstoff enthält, abtrennt (zurückgewinnt).

12. Verfahren zur Herstellung eines partikulären Vektors nach Anspruch 10, dadurch gekennzeichnet, daß man

a) mindestens einen sauren ionisierbaren Wirkstoff in einer vernetzten hydrophilen Matrix, die mit basischen ionischen Liganden bepfropft ist, bei einem pH-Wert oberhalb des pKa-Wertes des Wirkstoffes einkapselt,

b) den pH-Wert des Mediums bis auf einen Wert unterhalb des pKa-Wertes des Wirkstoffes herabsetzt und

c) die hydrophile Matrix, die den Wirkstoff hauptsächlich im Zentrum der genannten Matrix lokalisiert enthält, abtrennt (zurückgewinnt).

13. Verfahren nach einem der Ansprüche 10 bis 12, dadurch gekennzeichnet, daß die hydrophile Matrix aus Polysacchariden oder Oligosacchariden besteht, die in natürlicher oder chemischer Weise vernetzt worden sind.

14. Verfahren nach einem der Ansprüche 10 bis 13, dadurch gekennzeichnet, daß man eine Schicht aus amphiphilen Verbindungen auf der in der Stufe (c) erhaltenen Matrix fixiert.

15. Partikulärer Vektor, wie er nach dem Verfahren nach einem der Ansprüche 10 bis 14 erhältlich ist.

16. Pharmazeutische Zusammensetzung, dadurch gekennzeichnet, daß sie einen partikulären Vektor nach einem der Ansprüche 1 bis 9 oder einen solchen, wie er nach dem Verfahren gemäß einem der Ansprüche 10 bis 14 erhältlich ist, und einen für ihre Verabreichung pharmazeutisch akzeptablen Träger enthält.

17. Kosmetische Zusammensetzung, dadurch gekennzeichnet, daß sie einen partikulären Vektor nach einem der Ansprüche 1 bis 9 oder einen solchen, wie er nach dem Verfahren gemäß einem der Ansprüche 10 bis 14 erhältlich ist, und kosmetologisch akzeptable Exzipienten enthält.

18. Nahrungsmittel-Zusammensetzung, dadurch gekennzeichnet, daß sie einen Vektor nach einem der Ansprüche 1 bis 9 oder einen solchen, wie er nach dem Verfahren gemäß einem der Ansprüche 10 bis 14 erhältlich ist, enthält.

19. Verwendung eines Vektors nach einem der Ansprüche 1 bis 9 oder eines solchen, wie er nach dem Verfahren gemäß einem der Ansprüche 10 bis 14 erhältlich ist, zur Herstellung eines Arzneimittels.

% mol. de PE-NBD/mol de phospholipides

FIG.1

FIG. 2

FIG. 3